# EUROPÄISCHE PATENTSCHRIFT

(11) **EP 0 295 200 B1**
(45) Veröffentlichungstag und Bekanntmachung des Hinweises auf die Patenterteilung: **07.01.1993**
(21) Anmeldenummer: 88730134.9
(22) Anmeldetag: 13.06.1988
(51) Int. Cl.: A61F 2/32, A61F 2/36

(54) **Endoprothese**
Endoprosthesis
Endoprothèse

(30) Priorität: 12.06.1987 DE 8708501 U; 22.01.1988 DE 3802239
(43) Veröffentlichungstag der Anmeldung: 14.12.1988
(73) Patentinhaber: Goymann, Volkmar, Prof. Dr. med., D-45239 Essen (DE); Kranz, Curt, Dr.-Ing., D-10825 Berlin (DE)
(72) Erfinder: Kranz, Curt, D-1000 Berlin 62 (DE); Anapliotis, Emmanuel, D-1000 Berlin 33 (DE); Goymann, Volkmar, Prof., D-4300 Essen 16 (DE)
(74) Vertreter: Christiansen, Henning, Dipl.-Ing.

(56) Entgegenhaltungen:
- EP-A- 0 065 481
- EP-A- 0 181 586
- DE-A- 2 356 464
- US-A- 4 287 617

## Beschreibung

Die Erfindung betrifft eine Endoprothese der im Oberbegriff des Anspruchs 1 angegebenen Art.

Es sind Hüftendoprothesen bekannt, die aus einem Prothesenkopf, Prothesenkragen und einem Prothesenschaft bestehen, der im Markraum des Femur eines Patienten unter Verwendung von Knochenzement oder zementlos verankert wird. Bei zementloser Verankerung des Prothesenschaftes im Markraum des Femur wird eine Schaftstruktur verwendet, die eine möglichst feste Verankerung im Markraum sicherstellt, wobei eine poröse Oberfläche das Einwachsen von spongiösem Material erleichtern und damit die Festigkeit der Verankerung des Prothesenschaftes erhöhen soll.

Eine aus der EP-A-0181586 bekannte massive, einstückige, eintreibbare Hüftgelenkprothese weist am proximalen Ende eine Mehrzahl von Rippen auf, die vom Schaftkern sich nach außen erstrecken und deren Rückenflächen gezahnt sind. Die einzelnen Rippen sind derart ausgerichtet, daß sie rechtwinklig oder auch winkelig zueinander stehen. Nachteilig bei dieser bekannten Hüftgelenkprothese ist aber, daß die in den Körper einzuschlagende Materialmenge nicht gering ist und daß eine nicht unerhebliche Menge an Spongiosa vor der Einbringung der Endoprothese entfernt werden muß.

Eine andere aus der US-A-4287617 bekannte eintreibbare, aber nicht mit Schneidkanten versehene Hüftgelenkendoprothese weist einen Prothesenschaft auf, welcher eine im Querschnitt offene Kontur aufweist und aus mehreren plattenförmig ausgebildeten Lamellen, die sich in Eintreibrichtung der Endoprothese erstrecken, besteht. Zur Verankerung dieses Prothesenschaftes im Markraum des Femur ist aber auch eine weitgehende Entfernung der Spongiosa der regio intertrochanterica erforderlich. Die Entfernung der Spongiosa ist aber als unphysiologische lokale Störung im Sinne einer Defektbildung anzusehen, da dadurch die Belastungs- und vor allem altersabhängigen Anspassungsmöglichkeiten beeinträchtigt werden.

Ein weiterer Nachteil bekannter Endoprothesen ist eine ungleichmäßige Kraftverteilung durch eine primär distale Einleitung der Kräfte in den distal der regio intertrochanterica gelegenen Oberschenkelanteil, wodurch der eigentlich dafür vorgesehene Knochenanteil aus der funktionellen Belastung genommen wird. Dies führt zu einer Atrophie und nicht zu dem erwünschten funktionellen Reiz für den Knochen, fixierend an die Endoprothese heranzuwachsen.

Die Folge beider Nachteile ist eine vorzeitige Lockerung der Endoprothese mit der Notwendigkeit einer Revisionsoperation. Eine solche Revisionsoperation stößt aber bei vielen Endoprothesen auf erhebliche Schwierigkeiten, da die Prothesenschäfte vieler Endoprothesen so geformt sind, daß ein Entfernen der Endoprothese nur mit einer erheblichen Zerstörung des Knochenmaterials möglich ist, was letztlich das Einwachsen einer neuen Endoprothese erschwert und zum Teil unmöglich macht.

Ein weiterer Nachteil bekannter Endoprothesen besteht darin, daß Standardmodelle für unterschiedliche Femurgrößen vorhanden sind, die aber nicht an die individuelle Form des proximalen Femur eines Patienten angepaßt sind, so daß die ungünstige Krafteinleitung vieler Endoprothesen noch verstärkt und die vorhandene Knochensubstanz nicht optimal genutzt wird.

Schließlich machen die bekannten Endoprothesen eine aufwendige Operationstechnik erforderlich, bei der es notwendig ist, den mit der Prothese zu versehenden Knochen zur Aufnahme des Prothesenschaftes vorzumeißeln oder vorzubohren. Dies muß mit größter Vorsicht geschehen, da weder die Knochenstruktur unnötig beschädigt werden noch eine zu große Aushöhlung geschaffen werden darf, in der der Prothesenschaft nicht ausreichend fest verankert werden kann. Außerdem ist eine erhebliche Zeitspanne zum Raspeln oder Aufbohren erforderlich, was das Infektionsrisiko erheblich erhöht. Auch ist die Eröffnung des Markkanals erforderlich und eine erhebliche Gefahr einer Zerstörung des Knochens infolge einer via falsa gegeben.

Der Erfindung liegt die Aufgabe zugrunde, eine Endoprothese der eingangs genannten Art zu schaffen, die die Erhaltung der Physiologie des Knochenmarkraumes einschließlich der intertrochantären Spongiosa und damit günstige anatomische Bedingungen bei Revisionsoperationen und optimale Anpassungsmöglichkeiten an altersbedingte Knochenveränderungen, eine vereinfachte Operationstechnik ohne die Notwendigkeit eines Vormeißelns oder Vorbohrens und eine optimale physiologische Krafteinleitung sicherstellt sowie eine vergrößerte Anlagefläche für das Anwachsen von Knochenmaterial und eine individuelle Anpassung an die Form des Femur eines Patienten ermöglicht. Diese Aufgabe wird mit dem kennzeichnenden Merkmal des Anspruchs 1 gelöst.

Die erfindungsgemäße Lösung ermöglicht das Anbringen einer Endoprothese unter Erhalt der spongiösen Strukturen, so daß die Stoffwechselbiologie nicht gestört und die Durchblutungsbedingungen nicht grundsätzlich geändert werden. Durch den Erhalt der Physiologie des Knochenmarkraumes einschließlich der intertrochantären Spongiosa werden günstige anatomische Bedingungen bei Revisionsoperationen und optimale Anpassungsmöglichkeiten an altersbedingte Knochenveränderungen geschaffen. Die erfindungsgemäße Endoprothese ermöglicht eine sichere Verankerung der mit entlang ihrer Peripherie verlaufenden Schneidkanten versehenen Lamellen, so daß die jeweils in die Spongiosa einzutreibenden, gestuften Lamellen der Endoprothese selbstschneidend sind und kein Vorschneiden oder Raspeln erforderlich machen. Wesentliche Voraussetzung für das Eintreiben des Prothesenschaftes ist, daß alle einzutreibenden Teilflächen des Systems der Endoprothese parallel angeordnet sind und parallel zur Oberschenkelschaftachse einzutreiben sind, d.h. senkrecht zum Querschnitt eingeschlagen werden, ohne daß zusätzliche Vorarbeiten notwendig sind.

Dieses Vorgehen bietet entscheidende operationstechnische Vorteile, da ein Raspeln oder Aufbohren nicht notwendig ist, was eine erhebliche Zeitersparnis und eine Minderung des Infektionsrisikos sowie die Vermeidung einer via falsa zur Folge hat.

Da die Anzahl und die Querschnittsform der plattenförmig ausgebildeten Lamellen beliebig sein kann, wird zum einen eine vergrößerte Anlagefläche für das Anwachsen von Knochenmaterial und zum Anderen die Voraussetzung für eine individuelle Anpassbarkeit der Endoprothese an die jeweilige knochenform mit geringen Mitteln ermöglicht.

Der erfindungsgemäßen Lösung liegt die Erkenntnis zugrunde, daß als Fixationsteil ein grob durchlässiger Fremdkörper eingebracht wird, wobei die Sponiosa nur beim Eintreiben einmal auseinandergeschnitten wird, so daß sie sich danach mit dem größten Teil der Schnittfläche wieder exakt adaptieren kann.

Das durch die Form der erfindungsgemäßen Endoprothese mögliche Anbringen der Prothese am Femur eines Patienten unter weitestgehender Schonung der Spongiosa schafft eine gesicherte physiologische, d.h. proximale Krafteinleitung, wobei die spongiösen Strukturen extrem festen Halt bieten und das Übertragen enormer Druckkräfte erlauben.

Die erfindungsgemäße intertrochantäre Lamellenprothese ermöglicht einen sicheren Aufsitz durch plane Auflage des Prothesenkragens auf der Corticalis des Resektionsschnittes am Schenkelhals und der hier vorliegenden spongiösen Struktur. Dabei ist es unerheblich, ob bei der Operation ein Horizontal- oder ein Schrägschnitt gewählt wird.

Eine weitere vorteilhafte Ausgestaltung der erfindungsgemäßen Lösung zeichnet sich dadurch aus, daß die Lamellen und/oder die Verbindungslamellen und/oder die Verlängerungsstege Bohrungen und/oder Durchbrüche aufweisen. Dadurch werden zusätzliche Freiräume zum Einwachsen spongiösen Materials geschaffen, so daß zum einen die komplex verzahnte Spongiosastruktur zur Weiterleitung der auftretenden Kräfte genutzt wird und andererseits eine optimale Verbindung der Endoprothese mit dem Knochen sichergestellt wird, so daß Lockerungen nicht oder nur in geringem Maße zu befürchten sind.

Bei einem Verfahren zur Herstellung einer Endoprothese der genannten Art, bei dem die ventralen und dorsalen Lamellen nach einer vom Knochen eines Patienten angefertigten Röntgenschablone geschnitten und die einzelnen Teilflächen individuell der Form des Schenkelhalses bzw. des Trochantermassivs angepaßt zusammengesetzt werden, ist eine optimale Anpassung an die jeweilige Knochenform und damit eine optimale Krafteinleitung bei sicherem Sitz unter optimaler Ausnutzung des vorhandenen Knochenmaterials sichergestellt.

Einer anderen vorteilhaften Weiterbildung der Erfindung betrifft eine Prothese der eingangs genannten Gattung in Form einr Tibiaplateau-Prothese.

Eine derartige Prothese ist aus der DE-OS 34 29 157 als zementlos implantierbares Schienbeinplateau-Implantat bekannt, das aus einer Metallplatte und einem darauf befestigten Kunststoff-Gleitlager und einer Einlaßplatte besteht, die an der von dem Gleitlager abgewandten Seite an der Metallplatte im wesentlichen senkrecht zur Metallplatte befestigt ist. Die Einlaßplatte dient bei der bekannten Prothese im wesentlichen dazu, trotz des zementlosen Implantierens auch in belasteter Beugestellung des Kniegelenkes ein Auskippen und/oder Verschieben des Tibiaplateaus zu verhindern. Zum Herstellen einer festen Verbindung zwischen Tibiaplateau und Schienbeinkopf wird eine Knochenschraube durch die Einlaßplatte in den Schienbeinkopf geschraubt, was jedoch ein erschwertes Anbringen des Tibiaplateaus mit sich bringt und darüber hinaus eine zusätzliche Belastung des Schienbeins darstellt.

Eine derartige Tibiaplateau-Prothese soll leicht anbringbar und sowohl verschiebe- als auch kippsicher mit dem Schienbein verbindbar sein.

Die Lösung gemäß dieser Weiterbildung ermöglicht es, ein Tibiaplateau in dorsal-ventraler Richtung anzubringen und dabei eine verschiebe- und kippsichere Befestigung des Tibiaplateaus sicherzustellen. Da die erfindungsgemäße Tibiaplateau-Prothese ventral angesetzt und in dorsaler Richtung in die Tibia eingetrieben wird, ist es lediglich erforderlich, die vorderen Kniegelenkbänder seitlich zu verlagern. Die durch die Platten gebildeten großen Flächen in Querrichtung verhindern ein Abkippen des Tibiaplateaus auch bei einer Kniebeugebelastung mit einem damit verbundenen Einwirken eines Biegemoments auf die Prothese, was bei bekannten Tibiaplateaus wegen der auf den vorderen Plateaubereich einwirkenden Zugbeanspruchung zu einem Lösen des Tibiaplateaus aus seiner vorderen Verbindung mit dem Knochenlager führt.

Bei einer vorteilhaften Ausgestaltung sind die dem Tibiaplateau entgegengesetzten Unterkanten der plattenförmigen Lamellen über eine oder mehrere plattenförmigen Verbindungslamellen miteinander verbunden sind, so daß die Lamellen mit der Verbindungslamelle ein U-förmiges oder schwalbenschwanzförmiges Befestigungselement bilden. Diese Ausgestaltung der erfindungsgemäßen Lösung erhöht die Stabilität der Befestigung des Tibiaplateaus und damit die Sicherheit gegenüber einem Verschieben oder Auskippen des Tibiaplateaus.

Bei einer weiteren vorteilhafte Ausgestaltung sind die Vorderkanten der Lamellen bzw. der Verbindungslamelle schneidenförmig ausgebildet, was das Eintreiben des Befestigungselementes in die Tibia erleichtert und zu einer geringstmöglichen Beschädigung der Tibia führt.

Zusätzliche in den Lamellen und Verbindungslamellen vorgesehene Perforationen ermöglichen ein Einwachsen von Knochengewebe in die Perforationen und damit eine ergänzende Stabilisierung des Tibiaplateaus.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
Figuren 1 bis 17 verschiedene Ausführungsformen einer intertrochantären Lamellenprothese mit im Querschnitt offener Kontur in Seitenansicht, in perspektivischer Ansicht und z.T. im Querschnitt,
Figuren 18 bis 20 eine tertrochantäre Lamellenprothese mit im Querschnitt geschlossener Kontur in Seitenansicht, im Querschnitt und in perspektivischer Ansicht,
Figur 21 eine schematische, perspektivische Ansicht der erfindungsgemäßen Tibiaplateau-Prothese;
Figur 22 eine Seitenansicht eines Kniegelenks mit auf dem Schienbeinkopf befestigter Tibiaplateau-Prothese und
Figur 23 eine Draufsicht auf ein Kniegelenk mit auf dem Schienbeinkopf befestigter Tibiaplateau-Prothese.

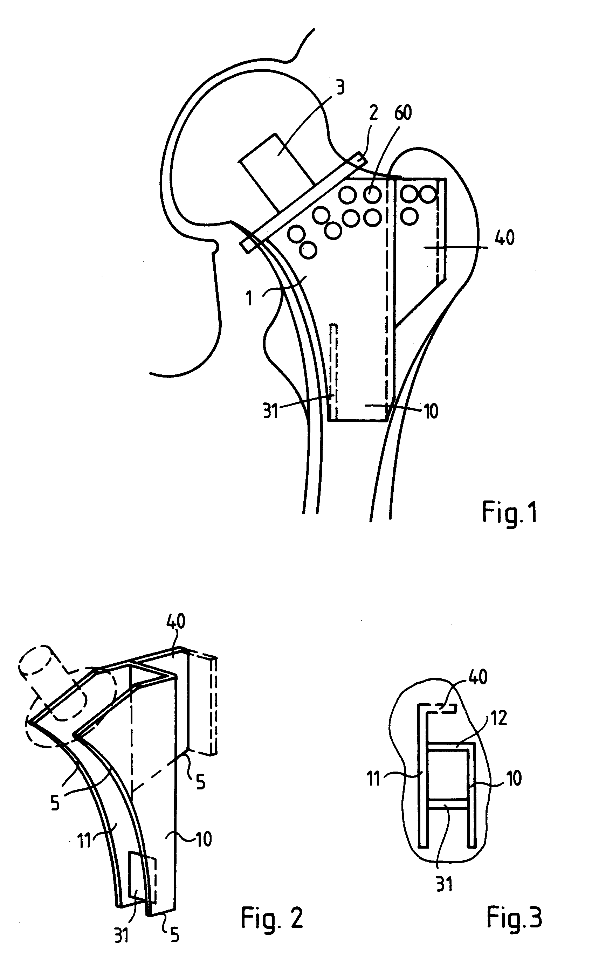

In den Figuren 1 bis 3 ist eine intertrochantäre Lamellenprothese in perspektivischer Ansicht, im Querschnitt und in Seitenansicht dargestellt, wobei die Seitenansicht schematisch in einen Femur eingezeichnet ist, um die Lage und die der Knochenform angepaßte Form der Endoprothese zu verdeutlichen.

Die Lamellenprothese besteht aus einem Prothesenschaft 1, einem Prothesenkragen 2 und einem konischen Zapfen 3 zur Aufnahme eines nicht näher dargestellten Prothesenkopfes. Der Prothesenschaft 1 weist zwei parallel zueinander angeordnete und als plattenförmige Teilflächen ausgebildete Lamellen 10, 11, 12 auf, deren periphere Kanten als Schneidkanten 5 ausgebildet sind. Eine der Schneidkanten 5 der Lamellen 10, 11 ist bogenförmig ausgebildet und damit der äußeren Form des Femur nachgebildet.

Die Lamelle 11 ist mit einem Verlängerungssteg 40 verbunden, wobei die Verbindung einstückig erfolgen kann. Auch der Verlängerungssteg 40 ist mit einer unteren Schneidkante 5 versehen.

Die beiden parallel zueinander angeordneten Lamellen 10, 11 sind über eine Verbindungslamelle 31 miteinander verbunden, die an ihrer Unterseite ebenfalls eine Schneidkante 5 aufweist.

Die Lamellen 10, 11, 12 und der Verlängerungssteg 40 ist mit Bohrungen 60 versehen, in die spongiöses Material einwachsen kann.

Die in den Figuren 1 bis 3 dargestellte, im Querschnitt U-förmige Lamellenprothese zeichnet sich durch eine größe Oberfläche zum An- und Einwachsen spongiösen Materials und durch eine optimale, der Knochenform angepaßte Form aus. Durch die in Längsrichtung des Prothesenschaftes 1 parallel verlaufenden Lamellen 10, 11, 12, die Verbindungslamelle 31 und den Verlängerungssteg 40 kann die Lamellenprothese senkrecht zur Femurachse eingesteckt bzw. eingeschlagen werden, ohne daß ein Vormeißeln oder Vorbohren notwendig ist.

Infolge der Schneidkanten 5 wird das Eintreiben der intertrochantären Lamellenprothese erleichtert. Auf diese Weise wird die normale Physiologie des Knochens voll erhalten und man hat bei einer eventuell notwendig werdenden Revisionsoperation noch ausreichend Knochensubstanz zur Verfügung, wobei die Resektion der Endoprothese bei einer Revisionsoperation wegen der parallel verlaufenden Teilflächen ohne wesentliche Beschädigung des Knochens erfolgt.

Die offene Strukturierung der in den Figuren 1 bis 3 dargestellten Endoprothese schafft die Voraussetzung dafür, daß die Prothese durch und durch mit Spongiosa durchwachsen werden kann, was ein Auslockern der Prothese auch bei starker und wechselnder Belastung verhindert.

Der lamellenförmige Aufbau der Prothese ermöglicht nicht nur eine individuelle Anpassung der Prothese an die jeweilige Anatomie des Knochens mit einfachen technischen Mitteln, da jeweils nur plattenförmige Teilflächen zu bearbeiten sind, sondern schafft zusätzlich auch die Voraussetzung dafür, daß die Lamellen und/oder Verbindungslamellen und/oder Verlängerungsstege so geformt werden können, daß unter Berücksichtigung der jeweiligen Anatomie des Knochens eine optimale Krafteinleitung und gleichmäßige Kraftverteilung ohne Druckkonzentrationen erfolgen kann.

Durch zusätzliche Verwendung eines bioverträglichen Materials mit Mikroporösität für die Lamellen und Verbindungsstege kann die Oberflächenstruktur weiter verbessert werden, so daß die Festigkeit des Sitzes der Endoprothese im Knochen erhöht wird.

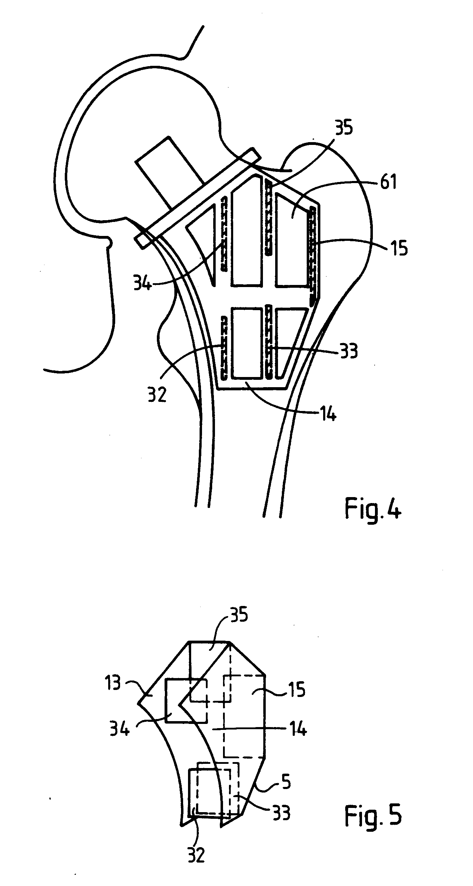

In den Figuren 4 und 5 ist eine Variante der intertrochantären Lamellenprothese dargestellt, die aus zwei parallel zueinander angeordneten Hauptlamellen 13, 14 und einer die parallelen Hauptlamellen 13, 14 verbindenden Lamelle 15 besteht. Zusätzlich sind die beiden Hauptlamellen 13, 14 durch Verbindungslamellen 32 bis 35 miteinander verbunden, die zur Vergrößerung der Oberfläche beitragen. Auch hier weisen die Lamellen periphere Schneidkanten 5 auf. Die Hauptlamellen 13, 14, 15 und ggf. die Verbindungslamellen 32 bis 35 sind mit Durchbrüchen 61 versehen, die das Durchwachsen spongiösen Materials ermöglichen.

Wie insbesondere der Seitenansicht gemäß Figur 4 zu entnehmen ist, ist diese Prothesenform der Knochenform optimal angepaßt, so daß eine maximale Oberfläche genutzt wird.

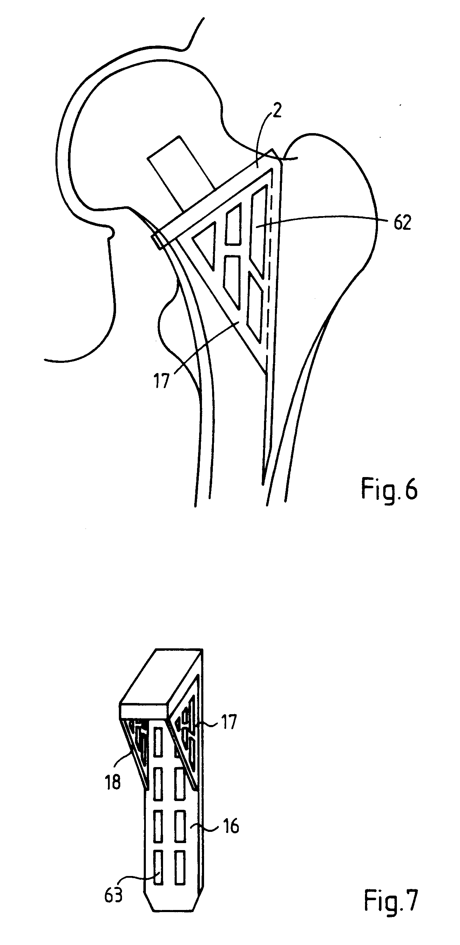

Figur 6 und 7 zeigt eine intertrochantäre Lamellenprothese mit einer plattenförmigen Hauptlamelle 16, die über zwei dreieckförmige seitliche Lamellen 17, 18 mit dem Prothesenkragen 2 verbunden ist. Die Hauptlamelle 16 ist an ihrem distalen Ende trapezförmig verjüngt ausgebildet und weist Durchbrüche 63 auf, die in dieser Ausführungsform rechteckförmig ausgebildet sind. Die in den dreieckförmigen Seitenlamellen 17, 18 vorgesehenen Durchbrüche 62 sind der äußeren Form der Seitenlamellen 17, 18 angepaßt.

Die Seitenansicht gemäß Figur 6 verdeutlicht die untere Schneidkante der Hauptlamelle 16, die ein leichtes Eintreiben der intertrochantären Lamellenprothese in den Femur ermöglicht.

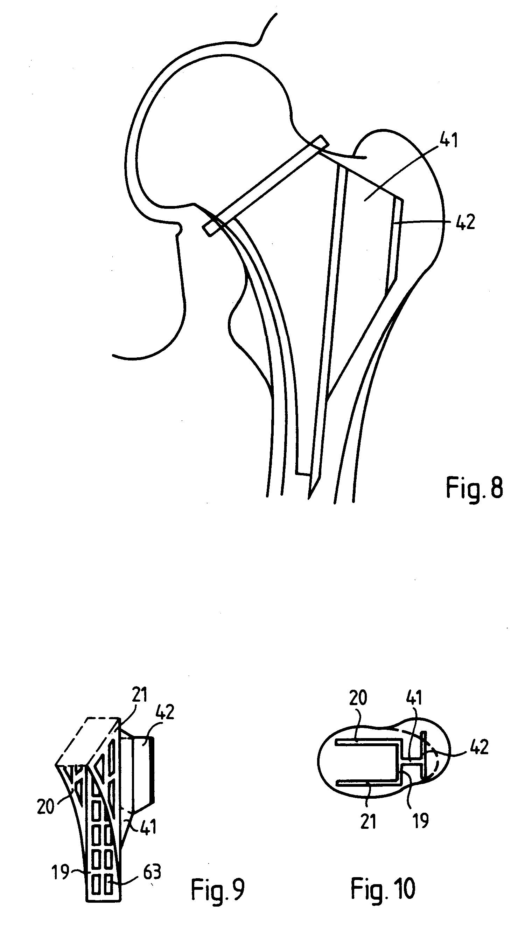

In den Figuren 8 bis 10 ist eine Variante der intertrochantären Lamellenprothese gemäß den Figuren 6 und 7 dargestellt.

Diese Prothese besteht ebenfalls aus einer Hauptlamelle 19 mit Durchbrüchen 63 und seitlich angesetzten Seitenlamellen 20, 21, deren periphere Schneidkanten bogenförmig der Knochenform angepaßt ausgebildet sind. Zusätzlich ist mittig an die Hauptlamelle 19 ein Verlängerungssteg 41 angesetzt, der rechtwinklig mit einer Verlängerungsplatte 42 verbunden ist, so daß eine T-förmige Verlängerung gegeben ist. Auch in dieser Ausführungsform sind die peripheren Kanten als Schneidkanten ausgebildet.

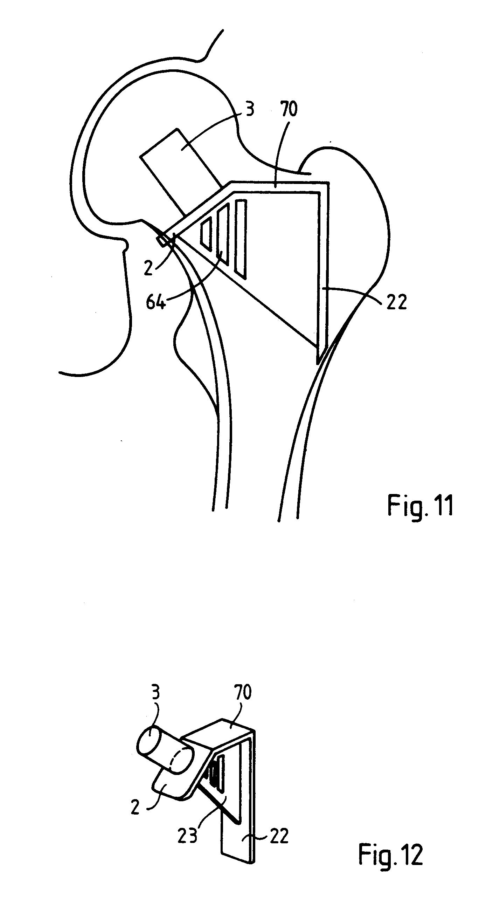

Die Figuren 11 und 12 zeigen in Seitenansicht bzw. in perspektivischer Ansicht eine intertrochantäre Lamellenprothese mit einer Hauptlamelle 22, die etwa im rechten Winkel mit einer oberen Deckfläche 70 verbunden ist, an die sich der Prothesenkragen 2 mit dem konisch geformten Zapfen 3 zur Aufnahme des Prothesenkopfes anschließt. Mittig auf die Hauptlamelle 22 aufgesetzt ist eine Lamelle 23, deren äußere Kanten mit der Deckfläche 70 und dem Prothesenkragen 2 verbunden sind. Die untere periphere Kante der Lamelle 23 ist ebenso wie die Unterkante der Hauptlamelle 22 als Schneidkante ausgebildet.

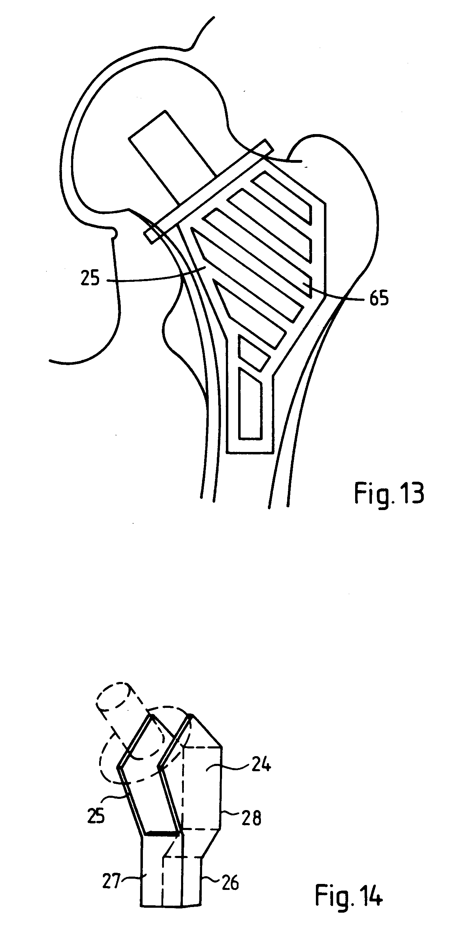

In den Figuren 13 und 14 ist in Seitenansicht bzw. in perspektivischer Ansicht eine kasten- oder trichterförmige intertrochantäre Lamellenprothese dargestellt. Sie besteht aus parallel zueinander verlaufenden Lamellen 24, 25 sowie die beiden Lamellen 24, 25 miteinander verbindenden, parallel zur Längsrichtung des Prothesenschaftes ausgerichtete Lamellen 26, 27, 28. Auch in dieser Ausführungsform sind die jeweils peripheren Kanten der Lamellen 24 bis 28 als Schneidkanten ausgebildet und mit Durchbrüchen 65 versehen, die der äußeren Geometrie der Lamellen angepaßt sind.

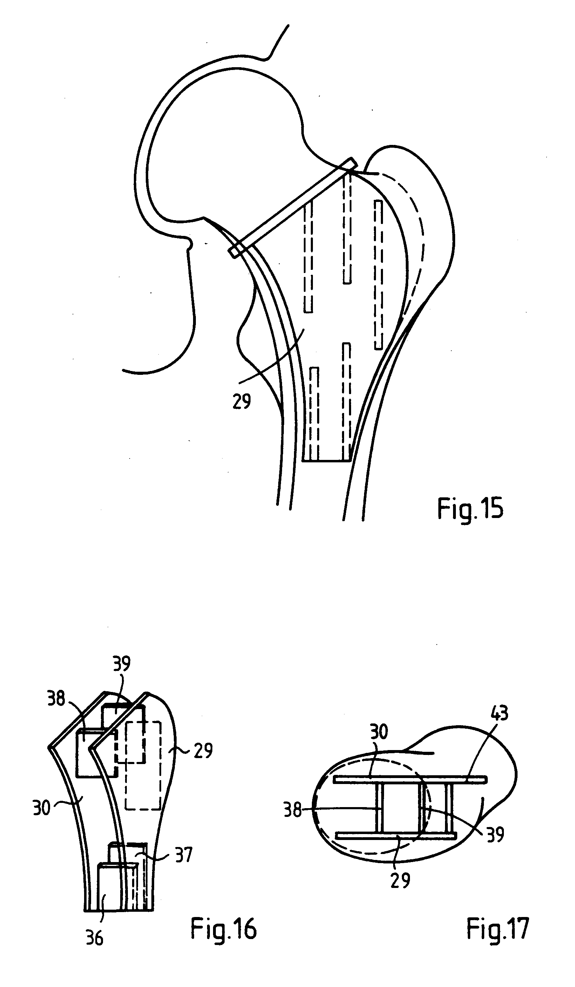

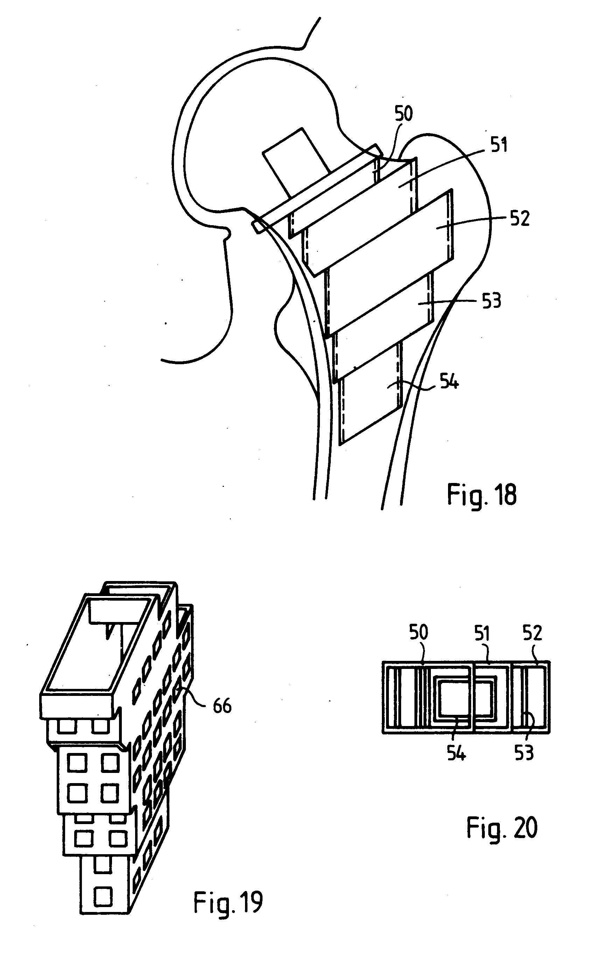

In den Figuren 15 bis 17 ist in Seitenansicht, perspektivischer Ansicht und im Querschnitt eine kasten- oder trichterförmige intertrochantäre Lamellenprothese dargestellt, die aus zwei parallel zueinander verlaufenden ventralen und dorsalen Lamellen 29, 30 mit dazwischen angeordneten, beide Lamellen 29, 30 miteinander verbindenden Verbindungslamellen 36 bis 39 besteht. Wie der Querschnittsdarstellung gemäß Figur 17 zu entnehmen ist, weist die eine Lamelle 30 einen verlängerten Steg 43 auf, der im implantierten Zustand im großen Rollhügel angeordnet ist.

In den Figuren 18 bis 20 ist eine intertrochantäre Lamellenprothese mit im Querschnitt geschlossener Kontur dargestellt, wobei diese Darstellung nur eine der möglichen geschlossenen Konturen ist. Die einzelnen Lamellen der kastenförmig gestaffelten intertrochantären Lamellenprothese weisen eine im Querschnitt rechteckförmige geschlossene Struktur auf und die einzelnen Elemente 50 bis 54 sind versetzt zueinander und der äußeren Kontur des Knochens angepaßt miteinander verbunden. Die jeweils herausragenden unteren peripheren Kanten sind als Schneidkanten ausgebildet. Zusätzlich weisen die Elemente 50 bis 54 Durchbrüche 66 auf, die das Einwachsen der Spongiosa ermöglichen.

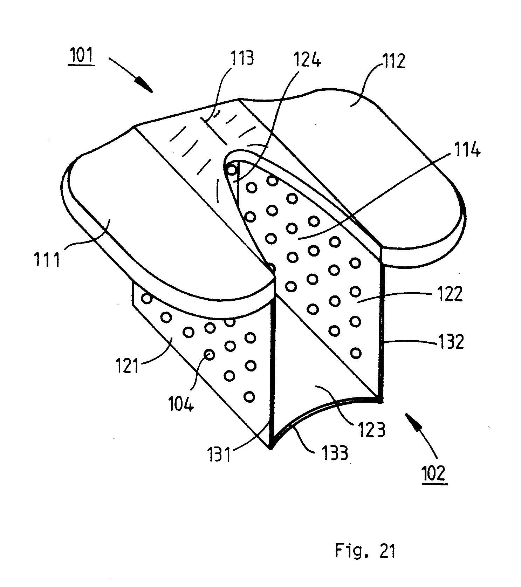

Die in Figur 21 dargestellte perspektivische Ansicht der erfindungsgemäßen Tibiaplateau-Prothese zeigt ein Tibiaplateau 101, das an seiner Oberseite zwei den Femur-Kondylenschalen entsprechende Stützflächen oder Gelenkpfannen 111, 112 aufweist, die in ventral-dorsaler Richtung konkav gekrümmt sind. Zwischen den Gelenkpfannen 111, 112 ist ein Mittelsteg 113 sowie ein Plateaueinschnitt 114 vorgesehen.

An der Unterseite des Tibiaplateaus 101, d.h. tibiaseitig sind zwei parallele plattenförmigen Lamellen 121, 122 in dorsal-ventraler Ausrichtung angebracht, deren Abstand zueinander etwa der breitesten Stelle des Einschnittes 14 des Tibiaplateaus entspricht. Die beiden parallelen Lamellen 121, 122 sind an ihren Unterkanten, d.h. ihren dem Tibiaplateau 101 entgegengesetzten Kanten mit einer ersten plattenförmigen Verbindungslamelle 123 miteinander verbunden. Eine zweite Verbindungslamelle 124 verbindet die Hinterkanten der beiden Lamellen 121, 122 sowie der ersten Verbindungslamelle 123.

Die Vorderkanten 131, 132, 133 der Lamellen 121, 122 sowie der ersten Verbindungslamelle 23 sind als Schneiden, d.h. scharfkantig ausgebildet, um ein Eintreiben der Tibiaplateau-Prothese in den Schienbeinkopf zu erleichtern. Zur besseren Führung sowie zur weiteren Erleichterung des Eintreibens der Prothese ist die Vorderkante 133 der ersten Verbindungslamelle 123 teilkreisförmig hinterschnitten.

Die parallelen Lamellen 121, 122 sowie gegebenenfalls die Verbindungslamellen 23, 24 weisen Perforationen 104 auf, die dazu dienen, nach dem Implantieren der Tibiaplateau-Prothese ein Einwachsen von Knochengewebe zu gestatten, um damit die Haltbarkeit und Stabilität der Tibiaplateau-Prothese zu erhöhen.

In Abweichung von dem in Figur 21 dargestellten Ausführungsbeispiel kann das aus den Lamellen 121, 122 und den Verbindungslamellen 123, 124 gebildete U-förmige Befestigungselement 102 auch als schwalbenschwanzförmiges Befestigungselement 102 ausgebildet sein, wobei die Lamellen 121, 122 vorzugsweise nach außen, d.h. in Richtung auf die Gelenkpfannen 111, 112 hin geneigt sind. Auch ist es grundsätzlich möglich, auf die Verbindungselemente 123, 124, zu verzichten, sofern sichergestellt ist, daß die Lamellen 121, 122 eine ausreichende Stabilität besitzen.

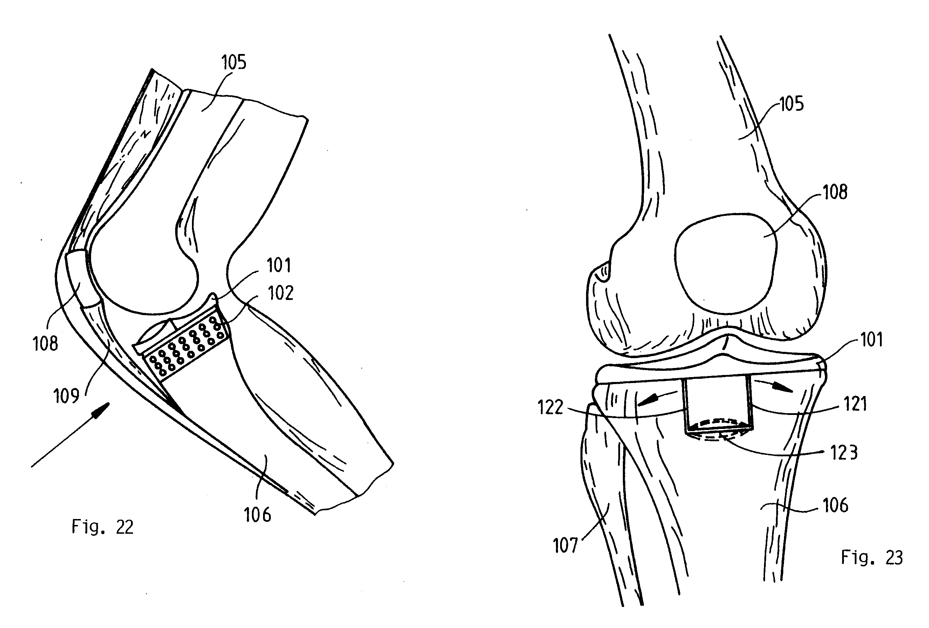

Die Funktionsweise sowie das Anbringen der erfindungsgemäßen Tibiaplateau-Prothese soll nachstehend anhand der Figuren 22 und 23 näher erläuter werden.

Figur 22 zeigt eine Seitenansicht eines Kniegelenks mit Femur 105, Tibia 106, Patella 108 sowie einen Querschnitt durch die vorderen Kreuzbänder 109. Die erfindungsgemäße Tibiaplateau-Prothese 101, 102 wird in Richtung des in Figur 22 schematisch eingetragenen Pfeiles nach dem Exponieren des Gelenkes und dem Entfernen der knorpeligen Flächen des Tibiaplateaus medial und lateral unter Mitnahme einer schmalen spongiösen subchondralen Knochenlamelle in den Tibiakopf eingetrieben, wobei die schneidenförmigen Vorderkanten der plattenförmigen Lamellen sowie der ersten Verbindungslamelle das Eintreiben der Prothese erleichtern. In der in Figur 22 dargestellten Endlage der Tibiaplateau-Prothese ist die obere Gleitfläche des Tibiaplateaus, die vorzugsweise aus Polyäthylen besteht, auf die Femurkondylen ausgerichtet, die im Falle einer Totalendoprothese ebenfalls als Implantat ausgeführt sind, während bei einer Teilendo-prothese die natürlichen Femurkondylen vorliegen.

Wie aus der Seitenansicht des Kniegelenks gemäß Figur 22 hervorgeht, sichern in dieser Lage der Tibiaplateau-Prothese 101, 102 die vorderen Kreuzbänder 109 zusätzlich die Prothese und verhindern in starker Beugestellung infolge ihres festen Anliegens am Schienbeinkopf jegliches Verschieben der Prothese in dorsalventraler Richtung, während die zweite Verbindungslamelle 124 ein Verschieben in ventral-dorsaler Richtung verhindert.

Die in Figur 23 dargestellte Vorderansicht des Kniegelenks gemäß Figur 22 zeigt neben Femur 105, Tibia 106, Patella 108, Fibula 107 und die im Schienbeinkopf befestigte Tibiaplateau-Prothese 101, 102.

Aus dieser Draufsicht geht deutlich hervor, wie die durch die Lamellen 121, 122 gebildeten großen Flächen in Querrichtung ein Abkippen der Prothese in Richtung der eingetragenen Pfeile verhindern. Die in Figur 23 als ebene Lamelle dargestellte erste Verbindungslamelle 123 kann wahlweise auch entsprechend der gestrichelten Darstellung einwärts oder auswärts gewölbt sein.

Die vorstehend beschriebenen intertrochantären Lamellen-prothesen zeichnen sich dadurch aus, daß die Druckkraftübertragung bzw. -weiterleitung in den Femurknochen proximal vom Hüftkopf mit seiner Spongiosa und von den spongiösen und cortikalen Anteilen des Schenkelhalses erfolgt. Dabei wird bei der Anbringung der Prothese die Physiologie des Markraumes einschließlich der intertrochanären Spongiosa erhalten, so daß eine biologische Anpassung im Rahmen der Alterung möglich ist. Im Gegensatz zu bekannten Prothesentypen, bei denen die Druckkraft am Calcar Femoris um 60% reduziert wird, was zu einer röntgenologisch nachweisbaren Resorption führt, wird bei den vorstehend beschriebenen Lamellenprothesen die Kraft physiologisch eingeleitet, wobei ein sicherer Aufsitz der Prothesen am Schenkelhals und eine optimale Verankerung im proximalen Femur sichergestellt ist.

Die Zusammensetzung der Prothesen aus einzelnen Lamellen ermöglicht es, mit einfachen technischen Mitteln jede Oberschenkelhalskonfiguration zu berücksichtigen und die Prothese entsprechend anzupassen. Dabei können unterschiedliche Krümmungen des Schenkelhalses und verschiedene Raumgrößen der regio intertrochanterica hinreichend berücksichtigt werden.

Zusätzlich führt die Lamellenform der Endoprothesen zu der Möglichkeit individuell anfertigbarer Prothesen nach einer Röntgenschablone, wobei die jeweils seitlichen Lamellen nach Röntgenschablonen geschnitten werden können. Außerdem ist ein baukastenartiges individuelles Zusammensetzen der Endoprothesen möglich.

Die jeweils peripheren, dem Prothesenkragen abgewandten Kanten der Lamellen sind als Schneidkanten ausgebildet, was zu einer extremen Vereinfachung der Operation führt und eine nahezu vollständige Erhaltung der natürlichen Knochensubstanz ermöglicht. Die Lamellen sind vorzugsweise aus Titanblech gefertigt, welches mit bekannten biokompatiblen Oberflächengestaltungen bzw. -beschichtungen versehen sein kann.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich anders gearteten Ausführungen Gebrauch machen.

## Patentansprüche

1. Zementlos zu verankernde Endoprothese, insbesondere mit einem in Längsrichtung in einem Knochen zu verankernden Prothesenschaft, wobei mehrere parallel angeordnete, miteinander verbundene, plattenförmige, eine im Querschnitt offene Kontur bildende Lamellen (10 bis 30, 121, 122) vorgesehen sind,
**dadurch gekennzeichnet,**
daß die Lamellen (10 bis 30, 121, 122) mit entlang ihrer Peripherie verlaufenden Schneidkanten (5, 131 bis 133) versehen sind.

2. Endoprothese nach Anspruch 1, **dadurch gekennzeichnet,** daß die Lamellen (10 bis 30, 121, 122) winkel- oder U-förmig miteinander verbunden sind.

3. Endoprothese nach Anspruch 1, **dadurch gekennzeichnet,** daß die Lamellen (10 bis 30) im Querschnitt dreieck-, rechteck-, trapez- oder vieleckförmig miteinander verbunden sind.

4. Endoprothese nach einem der vorangehenden Ansprüche, **dadurch gekennzeichnet,** daß bei Ausgestaltung als Schaftprothese die Außenkontur der Lamellen (10 bis 30) der Kontur des den Prothesenschaft (1) aufnehmenden Knochens angepaßt ist.

5. Endoprothese nach einem der vorangehenden Ansprüche, **gekennzeichnet durch** mindestens eine die Lamellen (10 bis 30, 121, 122) miteinander verbindende, plattenförmige Verbindungslamelle (31 bis 39, 123, 124).

6. Endoprothese nach Anspruch 5, **dadurch gekennzeichnet,** daß bei Ausgestaltung als Schaftprothese mehrere gegeneinander in Längsrichtung des Prothesenschaftes (1) versetzt zueinander angeordnete Verbindungslamellen (31 bis 39) vorgesehen sind.

7. Endoprothese nach Anspruch 5, **dadurch gekennzeichnet,** daß bei Ausgestaltung als Schaftprothese ein Teil der Verbindungslamellen (31 bis 39) in Längsrichtung des Prothesenschaftes (1) relativ zu anderen in gedrehter Position angeordnet ist.

8. Endoprothese nach einem der vorangehenden Ansprüche, **gekennzeichnet durch** mit den Lamellen (10 bis 30) verbundenen Verlängerungsstegen (40 bis 43).

9. Endoprothese nach einem der vorangehenden Ansprüche, **dadurch gekennzeichnet,** daß bei Ausgestaltung als Schaftprothese mehrere zu einer im Querschnitt offenen oder geschlossenen Kontur zusammengesetzte Lamellen (10 bis 30) als Elemente (50 bis 54) in Längsrichtung des Prothesenschaftes (1) miteinander verbunden und gegeneinander versetzt angeordnet sind.

10. Endoprothese nach einem der vorangehenden Ansprüche, **dadurch gekennzeichnet,** daß die Lamellen (10 bis 30, 121, 122) und/oder Verbindungslamellen (31 bis 39, 123, 124) und/oder Verlängerungsstege (40 bis 43) Bohrungen und/oder Durchbrüche (60 bis 66, 104) aufweisen.

11. Endoprothese nach einem der vorangehenden Ansprüche, **dadurch gekennzeichnet,** daß die Lamellen (10 bis 30, 121, 122) und/oder Verbindungslamellen (31 bis 39, 123, 124) und/oder Verlängerungsstege (40 bis 43) aus einer stabilen Drahtgitterkonstruktion bestehen.

12. Endoprothese nach einem der vorangehenden Ansprüche, **dadurch gekennzeichnet,** daß die Lamellen (10 bis 30, 121, 122) und/oder Verbindungslamellen (31 bis 39, 123, 124) und/oder Verlängerungsstege (40 bis 43) aus einem mikroporösen, bioverträglichen Material, insbesondere Titan, bestehen.

13. Endoprothese nach einem der vorangehenden Ansprüche, **dadurch gekennzeichnet,** daß bei der Ausgestaltung als Schaftprothese der Prothesenschaft (1) aus mehreren parallel zur Längsrichtung des Prothesenschaftes (1) angeordneten und miteinander verbundenen plattenförmigen Lamellen (10 bis 30) mit dem Prothesenkragen (2) abgewandten peripheren Schneidkanten (5) besteht.

14. Endoprothese nach Anspruch 1, **dadurch gekennzeichnet,** daß bei der Ausgestaltung als Tibiaprothese mit Gelenkpfannen zur Aufnahme der Femurkondylen auf der von den Gelenkpfannen (111, 112) abgewandten Seite des Tibiaplateaus (101) von diesem abstehend, in dorsal-ventraler Richtung parallel beabstandet zueinander verlaufend zwei dünne, in die Tibia (106) eingreifende Lamellen (121, 122) vorgesehen sind.

15. Endoprothese nach Anspruch 14, **dadurch gekennzeichnet,** daß die Lamellen (121, 122) im wesentlichen senkrecht auf dem Tibiaplateau (101) angeordnet sind und zusammen mit der ersten Verbindungslamelle (123) ein U-förmiges Befestigungselement (102) bilden.

16. Endoprothese nach Anspruch 14, **dadurch gekennzeichnet,** daß die Lamellen (121, 122) gegenüber dem Tibiaplateau (101) geneigt sind und zusammen mit der ersten Verbindungslamelle (123) ein schwalbenschwanzförmiges Befestigungselement bilden.

17. Endoprothese nach einem Ansprüche 14 bis 16, **dadurch gekennzeichnet,** daß die Vorderkanten (131, 132, 133) der Lamellen (121, 122) und der ersten Verbindungslamelle (123) als Schneiden ausgebildet sind.

## Claims

1. Endoprosthesis to be anchored without cement, more particularly having a prosthesis shank to be anchored longitudinally in a bone, a number of parallel, interconnected, plate shaped blades (10-30,121,122) being provided which form an open contour in cross section, characterised in that the blades (10-30,121, 122) are provided with cutting edges (5,131-133) extending along their periphery.

2. Endoprosthesis according to claim 1, characterised in that the blades (10-30,121,122) are interconnected in an angular or U-shaped configuration.

3. Endoprosthesis according to claim 1, characterised in that the blades (10-30) are connected to one another in a triangular, rectangular, trapezoidal or polygonal configuration in cross section.

4. Endoprosthesis according to one of the preceding claims, characterised in that, when it is constructed as a shank prosthesis, the outer contour of the blades (10-30) is adapted to the contour of the bone which receives the shank (1) of the prosthesis.

5. Endoprosthesis according to one of the preceding claims, characterised by at least one plate shaped connecting blade (31-39,123,124) which connects the blades (10-30,121,122) to one another.

6. Endoprosthesis according to claim 5, characterised in that, when it is in the form of a shank prosthesis, a plurality of connecting blades (31-39) are provided which are offset relative to one another in the longitudinal direction of the shank (1) of the prothesis.

7. Endoprosthesis according to claim 5, characterised in that, when the prosthesis is in the form of a shank prosthesis some of the connecting blades (31-39) are arranged in a rotated position relative to others in the longitudinal direction of the prosthesis shank (1).

8. Endoprosthesis according to one of the preceding claims, characterised by extending strips (40-43) connected to the blades (10-30).

9. Endoprosthesis according to one of the preceding claims, characterised in that, when it is in the form of a shank prosthesis, a plurality of blades (10-30) assembled to form an open or closed contour in cross section are connected to one another and offset relative to one another as elements (50-54) in the longitudinal direction of the prosthesis shank (1).

10. Endoprosthesis according to one of the preceding claims, characterised in that the blades (10-30,121,122) and/or connecting blades (31-39,123,124) and/or extending strips (40-43) are provided with bore holes and/or openings (60-66,104).

11. Endoprosthesis according to one of the preceding claims, characterised in that the blades (10-30,121,122) and/or connecting blades (31-39,123,124) and/or extending strips (40-43) are made of a stable wire mesh construction.

12. Endoprosthesis according to one of the preceding claims, characterised in that the blades (10-30,121,122) and/or connecting blades (31-39,123,124) and/or extending strips (40-43) consist of a microporous, biologically acceptable material, especially titanium.

13. Endoprosthesis according to one of the preceding claims, characterised in that, when it takes the form of a shaft prosthesis, the prosthesis shaft (1) consists of a plurality of plate shaped blades (10-30) arranged parallel to the longitudinal direction of the prosthesis shaft (1) and connected to one another, with peripheral cutting edges (5) facing away from the collar (2) of the prosthesis.

14. Endoprosthesis according to claim 1, characterised in that, when it takes the form of a tibia prosthesis with hinge socket for receiving the condyles of the femur on the side of the tibia plateau (101) remote from the joint socket (111,112), projecting therefrom, in the dorsal-ventral direction, extending at a parallel spacing from one another, there are two thin blades (121,122) engaging in the tibia (106).

15. Endoprosthesis according to claim 14, characterised in that the blades (121,122) are arranged substantially perpendicularly on the tibia plateau (101) and together with the first connecting blades (123) form a U-shaped fixing element (102).

16. Endoprosthesis according to claim 14, characterised in that the blades (121,122) are inclined relative to the tibia plateau (101) and together with the first connecting blade (123) form a dove tailed fixing element.

17. Endoprosthesis according to one of claims 14 to 16, characterised in that the front edges (131,132,133) of the blades (121,122) and the first connecting blade (123) are constructed as cutting edges.

## Revendications

1. Endoprothèse à ancrer sans ciment, en particulier avec un fût de prothèse à ancrer en direction longitudinale dans un os, prothèse dans laquelle sont prévues plusieurs lamelles (10 à 30, 121, 122) disposées parallèlement, reliées l'une à l'autre, en forme de plaque et formant un contour ouvert en coupe,
endoprothèse caractérisée
par le fait que les lamelles (10 à 30, 121, 122) présentent des arêtes de coupe (5, 131 à 133) courant le long de leur périphérie.

2. Endoprothèse selon la revendication 1, caractérisée par le fait que les lamelles (10 à 30, 121, 122) sont reliées l'une à l'autre sous un certain angle ou en forme de U.

3. Endoprothèse selon la revendication 1, caractérisée par le fait que les lamelles (10 à 30) sont reliées l'une à l'autre en formant, en coupe, un triangle, un rectangle, un trapèze ou un polygone.

4. Endoprothèse selon l'une des revendications précédentes, caractérisée par le fait qu'en cas de réalisation sous forme de prothèse à fût, le contour extérieur des lamelles (10 à 30) est adapté au contour de l'os qui reçoit le fût (1) de la prothèse.

5. Endoprothèse selon l'une des revendications précédentes, caractérisée par au moins une lamelle de liaison (31 à 39, 123, 124) en forme de plaque, reliant les lamelles (10 à 30, 121, 122) les unes aux autres.

6. Endoprothèse selon la revendication 5, caractérisée par le fait qu'en cas de réalisation sous forme de prothèse à fût, sont prévues plusieurs lamelles de liaison (31 à 39) disposées l'une à côté de l'autre, décalées l'une par rapport à l'autre selon la direction longitudinale du fût de la prothèse (1).

7. Endoprothèse selon la revendication 5, caractérisée par le fait qu'en cas de réalisation sous forme de prothèse à fût, une partie des lamelles de liaison (31 à 39) est disposée en position de rotation relative par rapport aux autres selon la direction longitudinale du fût de la prothèse (1).

8. Endoprothèse selon l'une des revendications précédentes, caractérisée par des barrettes de prolongement (40 et 43) liées aux lamelles (10 à 30).

9. Endoprothèse selon l'une des revendications précédentes, caractérisée par le fait qu'en cas de réalisation sous forme de prothèse à fût, plusieurs lamelles (10 à 30), assemblées pour donner un contour ouvert ou fermé en coupe, sont reliées les unes aux autres en tant qu'éléments (50 à 54), selon la direction longitudinale du fût de la prothèse (1) et sont disposées décalées les unes par rapport aux autres.

10. Endoprothèse selon l'une des revendications précédentes, caractérisée par le fait que les lamelles (10 à 30, 121, 122) et/ou les lamelles de liaison (31 à 39, 123, 124) et/ou les barrettes de prolongement (40 à 43) présentent des perçages et/ou des découpes (60 à 66, 104).

11. Endoprothèse selon l'une des revendications précédentes, caractérisée par le fait que les lamelles (10 à 30, 121, 122) et/ou les lammelles de liaison (31 à 39, 123, 124) et/ou les barrettes de prolongement (40 à 43) sont constituées d'une construction en toile métallique rigide.

12. Endoprothèse selon l'une des revendications précédentes, caractérisée par le fait que les lamelles (10 à 30, 121, 122) et/ou les lamelles de liaison (31 à 39, 123, 124) et/ou les barrettes de prolongement (40 à 43) sont constituées d'un matériau microporeux biocompatible, en particulier du titane.

13. Endoprothèse selon l'une des revendications précédentes, caractérisée par le fait qu'en cas de réalisation sous forme de prothèse à fût, le fût (1) de la prothèse est constitué de plusieurs lamelles en forme de plaque (10 à 30) disposées parallèlement à la direction longitudinale du fût (1) de la prothèse et reliées entre elles, avec des arêtes de coupe périphériques (5) du côté opposé au collet (2) de la prothèse.

14. Endoprothèse selon la revendication 1, caractérisée par le fait qu'en cas de réalisation sous forme de prothèse de tibia avec cavité articulaire pour recevoir les condyles du fémur sont prévues, du côté du plateau du tibia (101) opposé aux cavités articulaires (111, 112) et venant en saillie depuis ce plateau, deux lamelles minces (121, 122) qui sont orientées dans la direction dorso-ventrale, qui sont disposées à une certaine distance l'une de l'autre et parallèlement l'une à l'autre et qui viennent en prise dans le tibia (106).

15. Endoprothèse selon la revendication 14, caractérisé par le fait que les lamelles (121, 122) sont disposées sensiblement perpendiculairement au plateau du tibia (101) et forment, avec la première lamelle de liaison (23), un élément de fixation en forme de U (102).

16. Endoprothèse selon la revendication 14, caractérisée par le fait que les lamelles (121, 122) sont inclinées par rapport au plateau du tibia (101) et forment, avec la première lamelle de liaison (123), un élément de fixation en forme de queue d'aronde.

17. Endoprothèse selon l'une des revendications 14 à 16, caractérisée par le fait que les arêtes avant (131, 132, 133) des lamelles (121, 122) et de la première lamelle de liaison (123) sont conçues sous forme d'arêtes de coupe.
